# EUROPÄISCHE PATENTANMELDUNG

(11) **EP 2 724 737 A1**
(43) Veröffentlichungstag der Anmeldung: **30.04.2014**
(21) Anmeldenummer: 12189679.9
(22) Anmeldetag: 24.10.2012
(51) Int. Cl.: A61M 1/28, A61M 39/08, F16L 11/11

(54) **Medizinische Vorrichtung mit strukturiertem Folienschlauch**

(71) Anmelder: PolyCine GmbH, 66578 Schiffsweiler (DE)
(72) Erfinder: Dröschel, Stefan, 66125 Saarbrücken (DE); Schmidtlein, Martin, 66113 Saarbrücken (DE)
(74) Vertreter: Rippel, Hans Christoph

(57) **Zusammenfassung**

Die Erfindung betrifft eine medizinische Vorrichtung, vorzugsweise zur Peritonealdialyse, umfassend einen zur Aufnahme von Flüssigkeiten geeigneten Behälter (3); damit verbunden ein Folienschlauch (2) mit einer Konnektionsstelle, wobei die Wandung des Folienschlauchs (2) durch Falten und/oder Wellen strukturiert ist.

## Beschreibung

Die Erfindung betrifft eine medizinische Vorrichtung umfassend einen zur Aufnahme von Flüssigkeiten geeigneten Behälter, damit verbunden ein Folienschlauch mit einer Konnektionsstelle und gegebenenfalls einen oder mehrere weitere mit dem Folienschlauch verbundene, zur Aufnahme von Flüssigkeiten geeignete Behälter. Des Weiteren betrifft die Erfindung eine Vorrichtung zur Peritonealdialyse.

Im Bereich der Medizintechnik ergibt sich häufig das Problem, eine Flüssigkeit, vorzugsweise unter sterilen Bedingungen, aus einem Behälter in einen anderen oder auch den menschlichen Körper zu leiten, beispielsweise im Rahmen einer Infusion.

Gegenwärtig erfolgt der Transport von Flüssigkeiten im medizinischen Bereich überwiegend durch spezielle Schläuche. Solche Schläuche sind kostenintensiv in der Herstellung und neigen häufig zum Knicken unter Verschluss des Schlauches ("Kinking").

Es besteht daher ein Bedarf für eine Vorrichtung zur Entnahme von Flüssigkeiten im medizinischen Bereich, welche die Nachteile bekannter Systeme überwindet, das heißt insbesondere kostengünstig herzustellen ist und nicht zum "Kinking" neigt. Aus WO 2006/125603 A1 ist eine medizinische Vorrichtung zur Verwendung bei der Peritonealdialyse bekannt, welche einen Folienschlauch umfasst.

Dabei wird unter dem Begriff "Folienschlauch", insbesondere hinsichtlich einer Abgrenzung gegenüber dem Begriff eines "Schlauches" verstanden, dass ein Folienschlauch - im Gegensatz zum Schlauch - nur Rückstellkräfte aufweist, die wesentlich geringer als die Gewichtskraft sind. Dies bedeutet, dass der Folienschlauch ohne den Gegendruck durch eine Füllung in sich zusammenfällt. Dem gegenüber weist ein Schlauch eine Rückstellkraft auf, welche nach einem Zusammendrücken des Schlauches dafür sorgt, dass der Schlauch wieder auf den im Wesentlichen ursprünglichen Durchmesser zurückkehrt. Darüber hinaus ist die Wandstärke eines Folienschlauches geringer als bei einem Schlauch.

Der aus der WO 2006/125603 A1 bekannte Folienschlauch weist eine Wandstärke von vorzugsweise zwischen 50 und 300 µm auf. Des Weiteren ist der Folienschlauch vorzugsweise so beschaffen, dass die Innenseite bei einer Wärmebehandlung mit für den medizinischen Bereich typischen Temperaturen, insbesondere einer Sterilisierungstemperatur von 121°C, nicht verklebt. Dazu ist die Innenfläche des Folienschlauches im Allgemeinen strukturiert, bevorzugt aufgeraut. Eine Strukturierung kann beispielsweise durch Prägen erfolgen, bevorzugt erfolgt ein Aufrauen bereits beim Extrudieren durch Wahl einer geeigneten Düsenform.

Aus DE10 2006 055 651 A1 ist ein Folienschlauch bekannt, der zur weiteren Stabilisierung eine zusätzliche Stabilisierungssehne aufweist. Die Stabilisierungssehne kann dabei aus dem gleichen oder einem anderen Material wie der Folienschlauch gefertigt sein und entweder lose in diesen eingelegt oder fest mit dessen Wandung verbunden sein.

In WO 2004/004611 A1 ist eine Leitungsvorrichtung zum Führen von Flüssigkeiten und die Verwendung der Leitung zur Verbindung eines Katheters mit einem Urinauffangbehälter beschrieben. Die Leitungsvorrichtung ist flexibel ausgeführt, jedoch stark genug um einem Knicken entgegenzuwirken. Zur Verbesserung des Flüssigkeitsflusses ist in einer Ausführungsform vorgesehen, die Leitung an mindestens einer Stelle mit einer Riffelung zu versehen. Durch die Riffelung wird Gas eingeschlossen, wodurch der Flüssigkeitsfluss durch die Leitung verbessert bzw. das Entstehen einer ununterbrochenen Flüssigkeitssäule vermieden wird.

Aus EP 1 023 882 ist eine Katheteranordnung mit einem Schlauch, der geriffelte Bereiche aufweist bekannt. Je nach Art der Riffelung werden Bereiche geschaffen, mit denen sich die Länge des Schlauchs einstellen lässt. Mit anderen, weichen Riffelungen, werden besonders flexible Bereiche geschaffen.

Nachteilig an den aus dem Stand der Technik bekannten Schläuchen ist, dass durch die bekannten Schläuche das Problem des "Kinking" nicht völlig beseitigt werden kann oder durch Verstärkungen des Schlauchs größere Materialmengen erforderlich sind.

Es wurde gefunden, dass sich die Neigung von Folienschläuchen in einer medizinischen Vorrichtung zum "Kinking" deutlich verringern lässt, wenn die Wandung der verwendeten Folienschläuche durch Falten und/oder Wellen strukturiert ist.

Gegenstand der Erfindung ist daher eine medizinische Vorrichtung umfassend:
a) einen zur Aufnahme von Flüssigkeiten geeigneten Behälter;
b) damit verbunden ein Folienschlauch mit einer Konnektionsstelle, und
c) gegebenenfalls einen oder mehrere weitere, mit dem Folienschlauch verbundene, zur Aufnahme von Flüssigkeiten geeignete Behälter,
wobei die Wandung des Folienschlauches durch Falten und/oder Wellen strukturiert ist.

Die Wandstärke des verwendeten Folienschlauches beträgt bevorzugt zwischen 50 µm und 500 µm, bevorzugt zwischen 100 µm und 400 µm, besonders bevorzugt beträgt die Wandstärke ca. 200 µm.

In einer Ausführungsform der Erfindung ist die Wandung des Folienschlauches, im Querschnitt entlang der Längsachse des Folienschlauches gesehen, zick-zack-förmig strukturiert, das heißt die Wandung ist in der Art einer Ziehharmonika gefaltet.

In einer weiteren Ausführungsform der Erfindung ist die Wandung des Folienschlauches, im Querschnitt entlang der Längsachse des Folienschlauches gesehen, sinusförmig gewellt, das heißt der Folienschlauch ist in der Art eines Wellschlauches ausgestaltet.

In einer weiteren Ausführungsform ist die Wandung des Folienschlauches im Querschnitt entlang der Längsachse gesehen gewellt, wobei sich in Längsrichtung des Folienschlauches gesehen konkave und konvexe Halbkreisabschnitte abwechseln. Diese Halbkreisabschnitte bilden umlaufende Ringe aus, die die Form des Folienschlauches stabilisieren.

Weitere dem Fachmann geläufige Ausgestaltungen der Wandung des Folienschlauchs sind ebenfalls denkbar. Insbesondere ist es möglich die Wandung so zu strukturieren, dass eine Längenänderung des Folienschlauches durch zusammenschieben bzw. auseinanderziehen der Struktur ermöglicht wird, wie es beispielsweise bei Knick-Trinkhalmen bekannt ist. Ein Folienschlauch, dessen Länge sich durch Zusammenschieben verringern lässt, verringert vorteilhaft den erforderlichen Verpackungsaufwand. Zudem ist der Folienschlauch im zusammengeschobenen Zustand stabil genug, um diesen ohne weitere stabilisierende Maßnahmen einer Dampfsterilisation zu unterziehen.

Je nach Ausführungsform kann die Wellung des Folienschlauches in Form von geschlossenen Ringen oder einer Spirale ausgeführt sein.

Die Strukturierung des Folienschlauchs kann beispielsweise direkt beim Extrudieren erfolgen, indem bei der Extrusion ein Corrugator eingesetzt wird, der dem Folienschlauch direkt nach dem Verlassen des Extruders eine gewellte Form aufprägt. Dabei wird durch den Extruder das Schlauchmaterial durch einen ringförmigen Düsenspalt gedrückt. Über die Größe des Düsenspalts lässt sich die Wandstärke des Folienschlauchs einstellen. In einem nächsten Schritt wird der Schlauch mit Druckluft aufgeblasen und gegen Formbacken gedrückt. Die Formbacken stellen die Negativform der herzustellenden Struktur dar. Über die Formbacken wird dem Schlauchmaterial ein Großteil der Energie entzogen, so dass dieses aushärtet und die gewünschte Form dauerhaft annimmt.

Der Folienschlauch weist eine oder, vorzugsweise, mehrere Schichten auf. Die Herstellung des Mehrschichtfolienschlauches kann auf dem Fachmann an sich bekannte Art und Weise erfolgen. Geeignet hierfür ist beispielsweise das in der Patentanmeldung DE-A 103 61 851 beschriebene Verfahren.

Geeignete Mehrschichtfolien weisen vorzugsweise bis zu 7 Folienschichten auf. Besonders bevorzugt sind Mehrschichtfolien mit 2 bis 5 Schichten, insbesondere 3 Schichten.

Für die jeweiligen Schichten geeignete Materialien sind beispielsweise Mischungen aus modifiziertem Polypropylen. Dabei kann das Polypropylen in Mischung mit synthetischen Kautschuken, beispielsweise SEBS- (Styrol/Ethylen-Butylen/Styrol), SEPS- (Styrol/Ethylen-Propylen/Styrol) und SIS-Kautschuken (Styrol/Isopren/Styrol), vorliegen. Die SEBS und SEPS Kautschuke sind gegebenenfalls mit einem Öl modifiziert. Auch Copolymere aus Polyethylen und Polypropylen sind bevorzugt.

Die Phasenmatrix-Polymere enthalten im Falle von Dreischichtfolien neben der Polypropylen-Matrix in der Innenschicht im Allgemeinen 10 bis 40 Gew.-%, in der Mittelschicht 20 bis 60 Gew.-% und in der Außenschicht 0 bis 40 Gew.-% an einer Phase, vorzugsweise einer Blockcopolymerphase.

Durch die Wahl geeigneter Mischungen der vorgenannten Kunststoffe in den jeweiligen Schichten können Folienmaterialien mit unterschiedlichen Eigenschaften erhalten werden.

In einer Ausführungsform der vorliegenden Erfindung ist der verwendete Folienschlauch aus mindestens drei Schichten ausgebildet, wobei die Eigenschaften der Innen- und Außenschicht je nach Verwendung des erfindungsgemäßen Folienschlauches eingestellt wird.

In einer Ausführungsform ist die Außenschicht eine Siegelschicht, die ein Homo- oder Copolymer auf Polypropylenbasis mit einem Schmelzpunkt von ungefähr 125 bis 150 °C enthält.

Wenn eine dreischichtige Folienschlauch-Struktur vorliegt, so fungiert als Mittelschicht vorzugsweise ein Polypropylen-Copolymer. Alternativ kann die Mittelschicht auch ein Random-heterophasiges Copolymer aus Polypropylen mit vorzugsweise einer der oben genannten Phasen umfassen, das dem Folieschlauch zur Erhöhung der Schlagzähmodifizierung zugesetzt wird. Die Mittelschicht weist vorzugsweise einen Schmelzpunkt von 121 bis 150 °C, besonders bevorzugt 124 bis 135 °C auf.

Die Innenschicht des Folienschlauches ist in einer Ausführungsform wie die Mittelschicht ausgebildet, wobei der Innenschicht insgesamt weniger Schlagzähmodifizierer zugesetzt werden kann, so dass sich bessere Schweißeigenschaften des resultierenden Folienschlauches ergeben. Vorzugsweise ist die Innenschicht aus einem Material aufgebaut, das als Hauptbestandteil ein Polypropylen-Polymer umfasst, dessen Schmelzpunkt größer als 135 °C ist.

In einer weiteren Ausführungsform ist die Innenschicht eine temperaturresistente Schicht, enthaltend ein Homo- oder Copolymer auf Polypropylenbasis mit einem Schmelzpunkt von 125 bis 161 °C.

Anstelle von Polypropylen kann als Basismaterial auch Polyethylen verwendet werden, wobei die Materialien dann eine entsprechend niedrigere Schmelztemperatur aufweisen.

In einer Ausführungsform ist der Folienschlauch frei von PVC.

In einer weiteren Ausführungsform der vorliegenden Erfindung ist der Folienschlauch frei von Weichmachern.

In einer weiteren Ausführungsform der vorliegenden Erfindung ist der Folienschlauch frei von PVC und Weichmachern.

Bei den Behältern (a) und gegebenenfalls (c) handelt es sich vorzugsweise um Beutel, beispielsweise um Schlauchbeutel.

In einer bevorzugten Ausführungsform ist der Folienschlauch durch Peelnähte und/oder Ventile in Kammern unterteilt.

In einer weiteren Ausführungsform sind je nach gewünschtem Verwendungszweck die Verbindungen verschließbar. Zum Verschließen eignen sich dabei beispielsweise trennbare Peelnähte, Ventile oder Klammern. Die Funktionen Auslauf und Einlauf werden daher vorzugsweise über trennbare Peelnähte, Ventile oder Klammern gesteuert.

Der Verbund zwischen dem zur Aufnahme von Flüssigkeiten geeigneten Behälter und dem Folienschlauch kann auf jede geeignete Weise erfolgen:

Beispielhaft sei hierfür das Verschweißen des verwendeten Folienschlauches mit dem Behälter genannt, wobei die Außenseite des Folienschlauches mit der Innenseite des Behälters oder aber die Innenseite des erfindungsgemäßen Folienschlauchs mit dem Behälter verschweißt ist. Der verwendete Folienschlauch kann dabei über Peelnähte oder Ventile abgetrennt sein.

Alternativ kann der Behälter auch über ein Portelement mit dem Folienschlauch verbunden werden. Das Portelement dient zum Herstellen einer Verbindung zwischen dem Folienschlauch und dem Behälter mittels Stecken. Das Portelement kann mit dem Beutel verschweißt oder ebenfalls gesteckt sein.

Damit die Flüssigkeit nicht unmittelbar nach dem Anschluss der Vorrichtung abfließt, kann die Verbindung zwischen einem weiteren Behälter und dem Folienschlauch zunächst verschlossen sein. Hierfür eignet sich neben den bereits genannten Verschlussarten auch eine Aufreißversiegelung, wie eine Peelnaht.

Des Weiteren ist es denkbar, das Portelement mit einem Abbrechzapfen auszustatten, wobei durch den Abbrechzapfen der Behälter zunächst verschlossen ist. Nach dem Herstellen der Verbindung zwischen dem Behälter und dem Folienschlauch, beispielsweise durch Stecken, kann der Behälter durch Abbrechen des Abbrechzapfens geöffnet werden, so dass Flüssigkeit aus dem Behälter in den Folienschlauch gelangen kann.

Die Vorrichtung besitzt in einer bevorzugten Ausführungsform am Folienschlauch eine zweite Konnektionsstelle, beispielsweise zum Peritoneum eines Patienten. Diese zweite Verbindung wird vorzugsweise mit einem Katheter verbunden, der beispielsweise in das Peritoneum des Patienten führt. Bei dem Katheter handelt es sich zum Beispiel um einen Dauerkatheter. Die Verbindung des verwendeten Folienschlauches für den Katheter ist vorzugsweise als ein Y-Stück oder ein T-Stück ausgebildet, der auf den Folienschlauch aufgebracht ist.

Ein auf dem Folienschlauch zum Anschluss an einen Katheter angebrachtes Y-Stück bzw. T-Stück sollte vorzugsweise folgende Strömungssequenzen ermöglichen:
(1) Flüssigkeit, beispielsweise eine Drainagelösung, sollte beispielsweise aus der Bauchhöhle eines Patienten in einen gegebenenfalls vorhandenen weiteren Behälter, beispielsweise einen leeren Drainagebeutel fließen können.
(2) Die Flüssigkeit, beispielsweise eine Dialysatflüssigkeit, sollte durch den Folienschlauch, gegebenenfalls den T-Stück bzw. gegebenenfalls das Y-Stück in den gegebenenfalls vorhandenen Behälter, beispielsweise einen Drainagebeutel, hindurchgespült werden, um die Leitung zu reinigen.
(3) Die Flüssigkeit, beispielsweise eine Dialysatflüssigkeit, sollte in die Bauchhöhle eines Patienten eingeführt werden.

Die erfindungsgemäße Vorrichtung umfasst am Folienschlauch darüber hinaus gegebenenfalls eine dritte Verbindung, die an einen weiteren Behälter angebunden sein kann.

Der Folienschlauch ist mit diesem weiteren Behälter, beispielsweise dem Drainagebeutel, vorzugsweise unter Einhaltung der Sterilitätsbedingungen verbunden.

Der Verbund zwischen dem Behälter und dem Folienschlauch kann dabei auf jede geeignete Weise erfolgen:

Beispielhaft sei hierfür das Verschweißen des Folienschlauches mit dem Behälter genannt, wobei - je nach verwendetem Material des Behälters und des Folienschlauches - der Folienschlauch entweder mit seiner Außenseite oder seiner Innenseite des Behälters verschweißt ist. Zur Trennung zum Lösungszweig ist vorzugsweise eine Peelnaht, ein Ventil oder eine Klemme vorgesehen.

Alternativ kann der Behälter, beispielsweise ein Drainagebeutel, auch über ein Portelement, welches mit einem Abbrechzapfen ausgestattet sein kann, mit dem Folienschlauch durch Stecken verbunden werden. In diesem Fall kann das Portelement mit dem Behälter und dem Folienschlauch als Zwischenglied auch verschweißt sein.

Bei einer Ausführungsform der Erfindung sind der Behälter zur Aufnahme einer Flüssigkeit und gegebenenfalls vorhandener weiterer Behälter aus biegsamen Kunststoffmaterialen hergestellt. Dabei handelt es sich bevorzugt um coextrudierte Mehrschichtfolien aus Kunststoffen, die auf Polyolefinen basieren.

Vorzugsweise sind die für die erfindungsgemäße Vorrichtung verwendeten Behälter frei von PVC und/oder Weichmachern.

Die erfindungsgemäße Vorrichtung wird vorzugsweise wie folgt hergestellt:

Die Vorrichtungskomponenten liegen als Schlauchfolie oder als randbeschnittene Schlauchfolie in Form von Rollenware vor. Portelemente und die Y-Stücke oder T-Stücke liegen als Einzelteile vor.

Die Beutelkontur(en) wird/werden mittels Schweißvorrichtung durch Erhitzen unter Druck unlösbar verschweißt. Dann wird der Folienschlauch oder das Portelement in die Beutelkontur eingeführt und ebenfalls mit der Beutelkontur unlösbar verschweißt.

Wenn ein Portelement verwendet wird, so wird dieses dann mit dem Folienschlauch durch Verschweißen oder durch Stecken unlösbar verbunden.

Gegebenenfalls werden Peelnähte (lösbare Verschweißungen) und/oder Ventile als Kammertrennung oder Funktionsvorgabe eingeschweißt.

Eine Differenzierung bzw. Steuerung, ob eine lösbare oder unlösbare Verschweißung erzeugt wird, kann durch Variation von Schweißtemperatur, Schweißdruck und/oder Schweißzeit erfolgen.

Die Konnektionsstelle, beispielsweise ein Patientenanschluss, kann in oder auf den Folienschlauch unlösbar verschweißt angebracht werden oder an den Folienschlauch gesteckt werden.

Die erfindungsgemäße Vorrichtung eignet sich zum Umfiillen von Flüssigkeiten aller Art im medizinischen Bereich, beispielsweise zur Infusion von Flüssigkeiten in den menschlichen Körper. Dabei ist der Behälter (a) vorzugsweise ein Infusionsbeutel. Die Flüssigkeiten sind beispielsweise Blut, Blutplasma oder wässrige isotone Lösungen mit dem Elektrolytgehalt des Blutserums, welche beispielsweise Medikamente oder Nährstoffe enthalten (beispielsweise zur parenteralen Ernährung).

Ebenso bevorzugt dient die Vorrichtung zur Entnahme von Körperflüssigkeiten wie Blut, Urin oder Wundflüssigkeit.

Der Behälter (a) kann erfindungsgemäß auch der menschliche Körper sein, beispielsweise bei der Verwendung der Vorrichtung zur Hämodialyse, wo den Patienten Blut entnommen, über eine Maschine zur Blutreinigung gelenkt und anschließend in den Körper zurückgefiihrt wird.

In einer bevorzugten Ausführungsform handelt es sich bei der erfindungsgemäßen Vorrichtung um eine Vorrichtung zur Peritonealdialyse.

Die Peritonealdialyse, auch Bauchfelldialyse genannt, ist eine Variante der künstlichen Blutwäsche. Bei gesunden Menschen filtern die Nieren Stoffe aus dem Blut, damit sie mit dem Urin ausgeschieden werden können. Wenn die Nieren nicht mehr in der Lage sind, die anfallenden Stoffwechselprodukte auszuscheiden, muss das Blut künstlich gereinigt werden.

Während bei der künstlichen Niere (Hämodialyse) das Blut außerhalb des Körpers mit einem speziellen Filter gereinigt wird, benutzt man bei der Peritonealdialyse das gut durchblutete Bauchfell des Patienten als körpereigene Filtermembran. Das Bauchfell kleidet die gesamte Bauchhöhle aus. Bei der Bauchfelldialyse lässt man mehrmals am Tag eine Dialyselösung in die Bauchhöhle fließen, welche die giftigen Stoffwechselprodukte aufnimmt.

Die Peritonealdialyse führt der Patient - im Gegensatz zur Hämodialyse - alleine zu Hause durch und kann den Zeitplan nach seinen Bedürfnissen entsprechend flexibel gestalten. Bei der Peritonealdialyse sind die Patienten bezüglich der Nahrungs- und Flüssigkeitsaufnahme weniger eingeschränkt als bei der Hämodialyse. Jedoch besteht durch den permanent in der Bauchhöhle liegenden Katheter das Risiko von Infektionen an der Austrittsstelle oder in der Bauchhöhle.

Bei der Peritonealdialyse wird das Bauchfell als Blutfilter eingesetzt. Das Bauchfell (Peritoneum) ist eine gut durchblutete, halbdurchlässige Membran, welche die Bauchhöhle auskleidet und viele Organe überzieht. Über einen Katheter wird Dialyseflüssigkeit in die Bauchhöhle eingefüllt. In dieser Dialyseflüssigkeit ist eine andere Konzentration an Substanzen, als im Blut. Nach dem Prinzip der Osmose werden diese Substanzen dem Blut entzogen und gelangen in die Bauchhöhle. Nach einigen Stunden wird die Dialyseflüssigkeit mit den Harnbestandteilen wieder aus der Bauchhöhle ausgelassen.

Bei der Peritonealdialyse füllt der Patient selbst 1,5 bis 3,01 einer sterilen Dialyselösung über einen Katheter in die Bauchhöhle, die das Bauchfell (Peritoneum) damit umspült. Die Substanzen, die ausgeschieden werden sollen, wandern vom Blut durch das Peritoneum in die Dialyselösung.

Eine weitere Aufgabe der Dialyse besteht darin, dem Körper überschüssiges Wasser zu entziehen - der Fachmann spricht von Ultrafiltration. Deshalb enthalten die meisten Dialyselösungen Glukose (Zucker). Durch einen einfachen osmotischen Vorgang wandert bei der Peritonealdialyse auch Wasser in die Dialyselösung und kann so entfernt werden.
Nach etwa vier bis fünf Stunden ist die Dialyselösung, das so genannte Dialysat, mit Giftstoffen gesättigt. Es wird aus dem Bauchraum über den Katheter abgelassen und durch frische Dialyselösung ersetzt.

Zur Durchführung der Peritonealdialyse gibt es verschiedene Möglichkeiten: Bei der kontinuierlichen ambulanten Peritonealdialyse (CAPD) wechseln die Patienten selbst etwa vier bis fünf Mal am Tag ihre Dialyselösung. Bei der automatischen Peritonealdialyse (APD) übernimmt ein Dialysegerät (Cycler) den automatischen Beutelwechsel über Nacht - so ist der Patient tagsüber noch unabhängiger und fühlt sich kaum eingeschränkt.

Die Peritonealdialyse entspricht weitgehend der natürlichen Arbeitsweise der Niere, da sie den Körper kontinuierlich und gleichmäßig entgiftet und entwässert. Der Patient muss daher generell mit weniger Nebenwirkungen rechnen. Während der Dialyse ist der Patient mobil und unabhängig und kann seiner gewohnten Tätigkeit und seinem Beruf nachgehen.

Bei den Verfahren zur kontinuierlichen ambulanten Peritonealdialyse bzw. CAPD werden im Allgemeinen zwei Behälter benötigt, um das Verfahren durchzuführen. Ein erster Behälter (Ablauf- oder Drainagebeutel) ist gemeinsam mit einem Schlauchstück vorgesehen, welches über einen Katheter an das Peritoneum eines Patienten anschließbar ist, um das Peritoneum zu entleeren. Ein zweiter Behälter (Lösungs- oder Dialysatbeutel) enthält ein Dialysat für die Zuführung zu dem Peritoneum eines Patienten. Nachdem die Flüssigkeit aus dem Peritoneum in den Ablaufbeutel abgelaufen ist, wird der Lösungsbeutel an den Patienten angeschlossen. Das Dialysat wird durch den Schlauch aus dem Lösungsbeutel an das Peritoneum des Patienten abgegeben. Darüber hinaus gibt es auch so genannte Singlebeutel-Systeme, die nur einen Lösungsbeutel und einen Verbindungsschlauch mit Patientenanschluss aufweisen. In diesem Fall wird nach erfolgter Dialyse die Lösung aus dem Peritoneum in den alten, zuvor entleerten Dialysatbeutel zurückgeführt. Dieses erfordert jedoch, dass die Peritonealvorrichtung nach Entleerung in das Peritoneum für die gesamte Dauer der Dialyse am Patienten verbleibt. Singlebeutel kommen auch für die APD in Frage, wobei das Dialysegerät die verbrauchten Dialysatlösungen entsorgt.

In einer bevorzugten Ausführungsform handelt es sich daher bei der erfindungsgemäßen Vorrichtung um eine Vorrichtung zur Peritonealdialyse, umfassend
(a) einen Dialysatbeutel;
(b) einen Folienschlauch mit Patientenanschluss (Konnektionsstelle) und
(c) gegebenenfalls einen Drainagebeutel.

Die Vorrichtung ist **dadurch gekennzeichnet, dass** die Wandung des Folienschlauchs durch Falten und/oder Wellen strukturiert ist.

Der Folienschlauch weist erste, zweite und gegebenenfalls dritte Verbindungen auf, wobei die erste Verbindung zwischen dem Folienschlauch und dem Dialysatbeutel vorgesehen ist, das Peritoneum eines Patienten über die zweite Verbindung an den Folienschlauch angebunden werden kann und die gegebenenfalls vorhandene dritte Verbindung zwischen dem gegebenenfalls vorhandenen Drainagebeutel und dem Folienschlauch vorgesehen ist.

Die erfindungsgemäße Vorrichtung kann dabei für die kontinuierliche ambulante Peritonealdialyse (CAPD) oder die automatische Peritonealdialyse (APD) verwendet werden.

Zu Beginn eines typischen Zyklus der kontinuierlichen ambulanten Peritonealdialyse wird die in der Peritonealhöhle befindliche Lösung aus der Peritonealhöhle entfernt. Falls die erfindungsgemäße Vorrichtung einen Drainagebeutel umfasst, so wird die Lösung in der Peritonealhöhle über den Katheter und den daran angeschlossenen erfindungsgemäßen Folienschlauch in den vorhandenen Drainagebeutel überführt. Zu diesem Zweck ist die Verbindung zwischen dem Dialysatbeutel und dem Folienschlauch vorzugsweise geschlossen. Nach der Drainage wird dann eine Verbindung zwischen dem Dialysatbeutel und der Peritonealhöhle des Patienten über den erfindungsgemäßen Folienschlauch ermöglicht, während gleichzeitig die Verbindung zwischen Peritonealhöhle und dem Drainagebeutel unterbunden wird.

Im Neuzustand des Systems, d.h. vor der Verwendung zur Peritonealdialyse, können sowohl die Beutel und, falls vorhanden, als auch der erfindungsgemäße Folienschlauch mit dem Dialysat gefüllt sein.

In einer weiteren bevorzugten Ausführungsform der Erfindung sind der Dialysatbeutel, gegebenenfalls der Drainagebeutel und der Folienschlauch der Vorrichtung integral miteinander verbunden.

Der Dialysatbeutel der erfindungsgemäßen Vorrichtung ist für die Aufnahme des Dialysats bestimmt. Bei der Dialysatflüssigkeit kann es sich beispielsweise um eine Glucoselösung für die Peritonealdialyse handelt.
Üblicherweise hat der Dialysatbeutel ein Volumen von 500 bis 5000 ml, vorzugsweise 1000 bis 3000 ml, insbesondere 1250 bis 2750 ml. Insbesondere für die Verwendung der erfindungsgemäßen Vorrichtung bei (Klein-)Kindern können die verwendeten Dialysatbeutel auch kleiner ausgeprägt sein. Ferner kann der Dialysatbeutel in mehrere, d.h. in mindestens zwei Kammern unterteilt sein, wobei zwischen den jeweiligen Kammern Ventile und/oder Peelnähte vorgesehen sind, die ein Mischen der Kompartimente vor der Anwendung für Lösungen ermöglichen, die gemischt nicht haltbar sind.

Der Dialysatbeutel wird während der Peritonealdialyse an einem Stativ derart aufgehängt, dass die Dialysatflüssigkeit unter der Einwirkung des hydrostatischen Drucks in Richtung auf die durch die Aufhängung des Dialysatbeutels bedingte oben liegende Verbindung des Folienschlauches abfließen kann. Daher ist an dem Dialysatbeutel vorzugsweise mindestens eine Aufhängeöffnung vorgesehen, die dazu dient, mit einem Stativ in Eingriff zu gelangen, so dass die Anordnung des Dialysatbeutels für die Verabreichung des Dialysats richtig orientiert ist. Der Dialysatbeutel selbst kann weiterhin zusätzlich mindestens einen Stutzen aufweisen, über den Wirkstoffe und/oder Medikamente dem Dialysatbeutel und damit der Dialysatflüssigkeit zugesetzt werden können.

An der oben liegenden Verbindung des Folienschlauches ist somit der Dialysatbeutel mit der Dialysatlösung angebunden. Der Folienschlauch ist mit dem Dialysatbeutel vorzugsweise unter Einhaltung der Sterilitätsbedingungen verbunden, da bei der Peritonealdialyse die dem Peritoneum zugeführte Dialysatlösung in unsterilem Zustand ansonsten zu einer Peritonitis führen kann.

Der Verbund zwischen dem Dialysatbeutel und dem Folienschlauch kann dabei auf jede geeignete Weise erfolgen:

Beispielhaft ist hierfür das Verschweißen des Folienschlauches mit dem Dialysatbeutel genannt, wobei die Außenseite des Folienschlauches mit der Innenseite des Dialysatbeutels oder aber die Innenseite des Folienschlauchs mit dem Beutel verschweißt ist. Der Folienschlauch kann dabei über Peelnähte oder Ventile abgetrennt sein.

Alternativ kann der Dialysatbeutel auch über ein oben beschriebenes Portelement mit dem Folienschlauch verbunden werden. In diesem Fall kann das Portelement mit dem Beutel und dem Folienschlauch als Zwischenglied verschweißt sein.

Damit die Dialysatflüssigkeit nicht unmittelbar nach dem Anschluss der Vorrichtung an den Patienten in dessen Peritoneum fließt, kann die Verbindung zwischen Drainagebeutel und Folienschlauch zunächst verschlossen sein. Hierfür eignet sich neben den bereits genannten Verschlussarten auch eine Aufreißversiegelung, wie eine Peelnaht.

### Kurze Beschreibung der Figuren

Es zeigen
Figur 1: eine schematische Darstellung der Vorrichtung zur Peritonealdialyse
Figur 2: eine erste Ausführungsform des Folienschlauches,
Figur 3: eine zweite Ausführungsform des Folienschlauches und
Figur 4: eine weitere Ausführungsform des Folienschlauches.

### Ausführungsformen der Erfindung

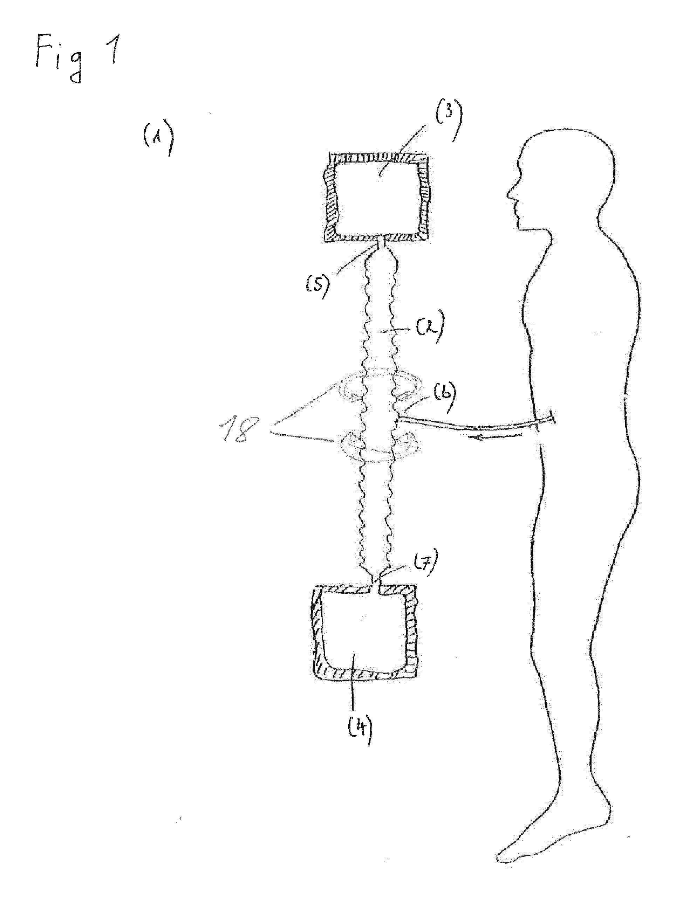

In Figur 1 ist eine Vorrichtung zur Peritonealdialyse schematisch dargestellt. Die Vorrichtung 1 umfasst einen Verbindungsfolienschlauch 2, erste 5, zweite 6 und dritte 7 Verbindungen. Die erste Verbindung 5 ist zwischen dem Verbindungs-Folienschlauch 2 und einem Dialysatbeutel 3 vorgesehen. Über die zweite Verbindung 6 an dem Verbindungs-Folienschlauch 3 ist eine Verbindung in das Peritoneum eines Patienten angebunden. Über die dritte Verbindung 7 wird eine Verbindung zum Drainagebeutel 4 hergestellt.

Für eine einfache und sichere Handhabung der Vorrichtung ist die Wandung des Verbindungs-Folienschlauches 3 strukturiert. Durch die Strukturierung werden die mechanischen Eigenschaften des Folienschlauches verbessert und einem "Kinking" entgegengewirkt. Zwei Ausführungsformen für die Strukturierung der Wandung des Folienschlauches sind in den nachfolgenden Figuren beschrieben.

Je nach Ausführungsform umfasst die Vorrichtung des Weiteren Klammern 18, mit denen der Folienschlauch 2 im Bereich der Verbindungen 5 und 7 abgedrückt werden kann. Dadurch kann ein Flüssigkeitsfluss gezielt ermöglicht oder unterbunden werden.

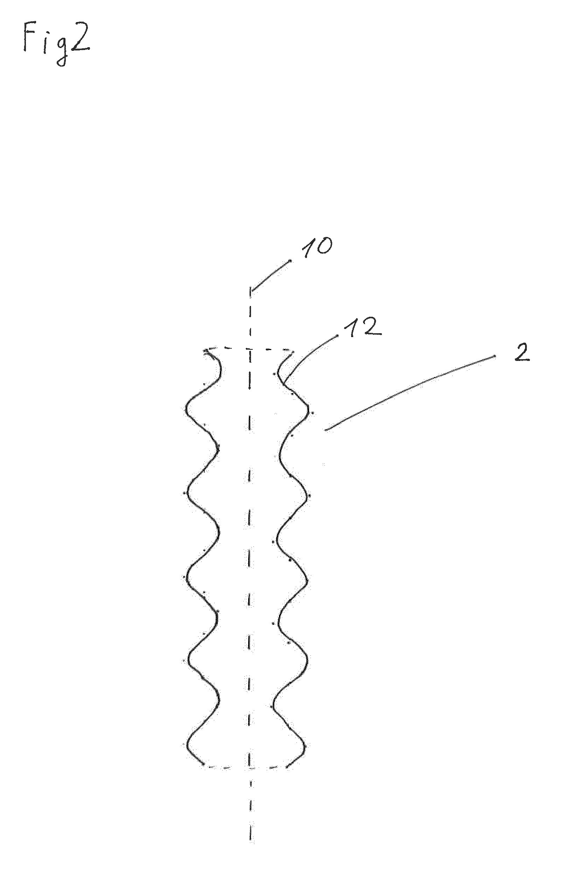

Figur 2 zeigt eine erste Ausführungsform des strukturierten Folienschlauches.

Figur 2 zeigt einen Folienschlauch 2 in einer Schnittdarstellung entlang der Längsachse 10 des Folienschlauches 2. Die Wandung des Folienschlauches scheint im Querschnitt entlang der Längsachse 10 sinusförmig gewählt. Der Folienschlauch 2 ist somit in der Art eines Wellschlauches ausgestaltet.

Je nach Ausführungsform kann die Wellung des Folienschlauches in Form von geschlossenen Ringen oder einer Spirale ausgeführt sein.

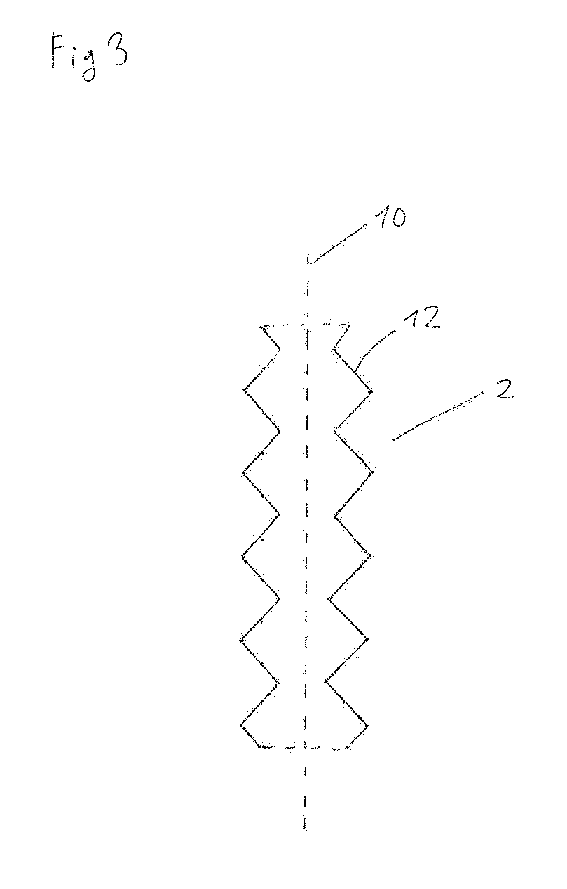

In Figur 3 ist eine zweite Ausführungsform des strukturierten Folienschlauches dargestellt.

Figur 3 zeigt den Folienschlauch 2 in einer Schnittdarstellung entlang der Längsachse 10 des Folienschlauches 2. Die Wandung 12 des Folienschlauches 2 erscheint im Querschnitt entlang der Längsachse 10 des Folienschlauches 2 zick-zack-förmig, das heißt der Folienschlauch 2 ist in der Art einer Ziehharmonika gefaltet.

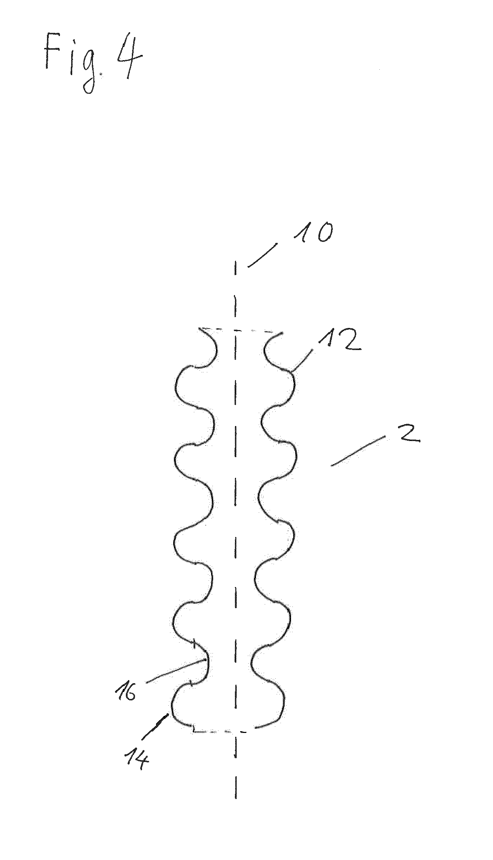

In Figur 4 ist eine weitere Ausführungsform des strukturierten Folienschlauches dargestellt.

Figur 4 zeigt einen Folienschlauch 2 in einer Schnittdarstellung entlang der Längsachse 10 des Folienschlauches 2. Die Wandung des Folienschlauches ist, im Querschnitt entlang der Längsachse 10 gesehen, gewellt, wobei sich in Längsrichtung des Schlauchs gesehen konkave 16 und konvexe 14 Halbkreisabschnitte abwechseln. Diese Halbkreisabschnitte bilden umlaufende Ringe aus, die die Form des Folienschlauchs 2 stabilisieren.

## Patentansprüche

1. Medizinische Vorrichtung, umfassend
(a) einen zur Aufnahme von Flüssigkeiten geeigneten Behälter (3);
(b) damit verbunden ein Folienschlauch (2) mit einer Konnektionsstelle und
(c) gegebenenfalls einen oder mehrere weitere, mit dem Folienschlauch (2) verbundene, zur Aufnahme von Flüssigkeiten geeignete Behälter (4), **dadurch gekennzeichnet, dass** die Wandung des Folienschlauchs (2) durch Falten und/oder Wellen strukturiert ist.

2. Vorrichtung nach Anspruch 1, **dadurch gekennzeichnet, dass** der Folienschlauch (2) eine Wandstärke zwischen 50 µm und 500 µm aufweist.

3. Vorrichtung nach Anspruch 1 oder 2, **dadurch gekennzeichnet, dass** die Wandung des Folienschlauchs (2), im Querschnitt entlang der Längsachse (10) des Folienschlauchs (2) gesehen, zickzack förmig, das heißt in der Art einer Ziehharmonika, gefaltet ist.

4. Vorrichtung nach Anspruch 1 oder 2, **dadurch gekennzeichnet, dass** die Wandung des Folienschlauchs (2), im Querschnitt entlang der Längsachse (10) des Folienschlauchs (2) gesehen, sinusförmig, das heißt in der Art eines Wellschlauchs, ausgestaltet ist.

5. Vorrichtung nach Anspruch 1 oder 2, **dadurch gekennzeichnet, dass** die Form der Wandung des Folienschlauchs (2), im Querschnitt entlang der Längsachse (10) des Folienschlauchs (2) gesehen, abwechselnd aus konvexen (14) und konkaven (16) Halbkreisabschnitten aufgebaut ist.

6. Vorrichtung nach einem der Ansprüche 1 bis 5, **dadurch gekennzeichnet, dass** der Folienschlauch (2) aus einem oder mehreren Polyolefinen besteht.

7. Vorrichtung nach Anspruch 5, **dadurch gekennzeichnet, dass** der Folienschlauch (2) aus mindestens einer Folienschicht besteht und die mindestens eine Folienschicht Copolymere aus Polyethylen und Polypropylen oder modifiziertes Polypropylen umfasst, wobei das Polypropylen in Mischung mit synthetischen Kautschuken, insbesondere Styrol/Ethylen-Butylen/Styrol-, Styrol/Ethylen-Propylen/Styrol- und Styrol/Isopren/Styrol-Kautschuken, vorliegt.

8. Vorrichtung zur Peritonealdialyse (1) gemäß einem der Ansprüche 1 bis 7, umfassend
(a) einen Dialysatbeutel (3),
(b) einen Folienschlauch (2) mit Patientenanschluss und
(c) gegebenenfalls einen Drainagebeutel (4),
**dadurch gekennzeichnet, dass** der Folienschlauch (2) erste (5), zweite (6) und gegebenenfalls dritte (7) Verbindungen aufweist, wobei die erste Verbindung (5) zwischen dem Folienschlauch (2) und dem Dialysatbeutel (3) vorgesehen ist, das Peritoneum eines Patienten über die zweite Verbindung (6) an den Folienschlauch (2) angebunden werden kann, und die gegebenenfalls vorhandene dritte Verbindung (7) zwischen dem gegebenenfalls vorhandenen Drainagebeutel (4) und dem Folienschlauch (2) vorgesehen ist.
